# EUROPEAN PATENT SPECIFICATION

(11) **EP 1 028 706 B1**
(45) Date of publication and mention of the grant of the patent: **09.07.2003**
(21) Application number: 98956518.9
(22) Date of filing: 03.11.1998
(51) Int. Cl.: A61K 9/00, A61K 47/18

(54) **BUFFERED DRUG FORMULATIONS FOR TRANSDERMAL ELECTROTRANSPORT DELIVERY**
GEPUFFERTE ARZNEISTOFFFORMULIERUNGEN ZUR TRANSDERMALEN VERABREICHUNG DURCH ELEKTROTRANSPORT
FORMULATIONS PHARMACEUTIQUES TAMPONNEES A ADMINISTRATION TRANSDERMIQUE PAR ELECTROTRANSPORT

(30) Priority: 12.11.1997 US 969217
(43) Date of publication of application: 23.08.2000
(73) Proprietor: ALZA CORPORATION, Palo Alto California 94303-0802 (US)
(72) Inventor: LEUNG, Iris, Ka, Man, Chesterfield, MO 63017-5514 (US); CORMIER, Michel, J., N., Mountain View, CA 94043 (US); SENDELBECK, Sara, Lee, Palo Alto, CA 94306-2631 (US); MUCHNIK, Anna, Belmont, CA 94002 (US)
(74) Representative: Campbell, Neil Boyd
(86) International application number: US9823411
(87) International publication number: WO99024015

(56) References cited:
- WO-A-93/12812
- WO-A-97/39768
- GREEN P G: "IONTOPHORETIC DELIVERY OF PEPTIDE DRUGS" JOURNAL OF CONTROLLED RELEASE, vol. 41, no. 1/02, 1 August 1996, pages 33-48, XP000592981

## Description

### Technical Field

The invention relates generally to drug formulations used in transdermal electrotransport drug delivery. More particularly, the invention relates to buffered drug formulations for transdermal electrotransport delivery using buffers which minimally compete with the drug for carrying electric current and which have greater stability and a longer shelf life.

### Background of the Invention

Transdermal (i.e., through the skin) delivery of therapeutic agents affords a comfortable, convenient and noninvasive technique for administering drugs. The method provides several advantages over conventional modes of drug delivery. For example, variable rates of absorption and (e.g., hepatic) metabolism encountered in oral treatment are avoided, and other inherent inconveniences -- e.g., gastrointestinal irritation and the like -- are eliminated. Transdermal delivery also allows a high degree of control over blood concentrations of a particular drug and is an especially attractive administration route for drugs with narrow therapeutic indexes, short half-lives and potent activities.

Transdermal delivery can be either passive or active. Many drugs are not suitable for passive transdermal drug delivery because of their size, ionic charge characteristics and hydrophilicity. One method of overcoming this limitation is the use of low levels of electric current to actively transport drugs into the body through skin. This technique is known as "electrotransport" or "iontophoretic" drug delivery. The technique provides a more controllable process than passive transdermal drug delivery since the amplitude, timing and polarity of the applied electric current is easily regulated using standard electrical components. In this regard, electrotransport drug flux can be from 50% to several orders of magnitude greater than passive transdermal flux of the same drug.

Electrotransport devices generally employ at least two electrodes. Both of these electrodes are positioned in intimate electrical contact with some portion of the skin of the body. One electrode, called the active or donor electrode, is the electrode from which the therapeutic agent is delivered into the body. The other electrode, called the counter or return electrode, serves to close the electrical circuit through the body. In conjunction with the patient's skin, the circuit is completed by connection of the electrodes to a source of electrical energy, e.g., a battery, and usually to circuitry capable of controlling the current applied by the device through the patient.

Depending upon the electrical charge of the species to be delivered transdermally, either the anode or cathode may be the active or donor electrode. Thus, if the ionic substance to be driven into the body is positively charged, the positive electrode (the anode) will be the active electrode and the negative electrode (the cathode) will serve as the counter electrode, completing the circuit. On the other hand, if the ionic substance to be delivered is negatively charged, the cathodic electrode will be the active electrode and the anodic electrode will be the counter electrode.
Alternatively, both the anode and the cathode may be used to deliver drugs of appropriate charge into the body. In this case, both electrodes are considered to be active or donor electrodes. In other words, the anodic electrode can deliver positively charged agents into the body while the cathodic electrode can deliver negatively charged agents into the body.

Existing electrotransport devices additionally require a reservoir or source of the therapeutic agent that is to be delivered into the body. Such drug reservoirs are connected to the anode or the cathode of the electrotransport device to provide a fixed or renewable source of one or more desired species or agents. Examples of reservoirs and sources include a pouch as described in U.S. Patent No. 4,250,878 to Jacobsen; a pre-formed gel body as disclosed in U.S. Patent No. 4,383,529 to Webster; and a glass or plastic container holding a liquid solution of the drug, as disclosed in the figures of U.S. Patent No. 4,722,726 to Sanderson et al.

Of particular interest herein is the transdermal delivery of peptides, polypeptides, and proteins because of the problems encountered with more common drug administration routes such as oral delivery. Polypeptide and protein molecules are highly susceptible to degradation by proteolytic enzymes in the gastrointestinal tract and are subjected to an extensive hepatic metabolism when taken orally. Thus, these substances usually require parenteral administration to achieve therapeutic levels in the patient's blood. The most conventional parenteral administration techniques are hypodermic injections and intravenous administration. Polypeptides and proteins are, however, inherently short acting in their biological activity, requiring frequent injections, often several times a day, to maintain the therapeutically effective levels needed. Patients frequently find this treatment regimen to be inconvenient and painful. Such therapy also includes risk of, e.g., infection.

Much effort has been expended to find other routes (other than parenteral injections) for effective administration of pharmaceutical agents, including polypeptides and proteins. Administration routes with fewer side effects as well as better patient compliance have been of particular interest. Such alternative routes have generally included "shielded" oral administration wherein the polypeptide/protein is released from a capsule or other container after passing through the low pH environment of the stomach, delivery through the mucosal tissues, e.g., the mucosal tissues of the lung with inhalers or the nasal mucosal tissues with nasal sprays, and implantable pumps. Unfortunately, these alternative routes of polypeptide/protein delivery have met with only limited success.

A number of investigators have disclosed electrotransport delivery of polypeptides and proteins. An early study by R. Burnette et al. J. Pharm. Sci. (1986) 75:738, involved in vitro skin permeation of thyrotropin releasing hormone, a small tripeptide molecule. The electrotransport flux was found to be higher than passive diffusional flux. Chien et al. J. Pharm. Sci. (1988) 78:376, in both in vitro and in vivo studies, showed that transdermal delivery of vasopressin and insulin via electrotransport was possible. See, also, Maulding et al., U.S. Statutory Invention Registration No. H1160, which discloses electrotransport delivery of calcitonin in minipigs.

However, transdermal delivery of polypeptide and protein drugs has also encountered technical difficulties. For example, skin irritation can occur due to water hydrolysis at the interface between the electrode and the drug solution or electrolyte salt solution. The products of such hydrolysis, hydronium ions at the anode and hydroxyl ions at the cathode, compete with drug ions of like charge for delivery into the skin, altering skin pH and causing irritation. U.S. Patent No. 5,533,971, to Phipps et al., describes this problem in more detail and reports the use of amino acid buffers, including histidine buffers, for adjusting the pH of electrotransport device reservoirs to levels which cause less irritation. Histidine as well as Asp, Glu and Lys have been used for buffering (U.S. Patent 5,624,415). Additionally, certain polypeptide and protein drugs, particularly those that are not native to the animal being treated, may cause skin reactions, e.g., sensitization or irritation. Many polypeptide and protein drugs are also unstable and degrade rapidly. In this regard, International Publication No. WO 93/12812, published 8 July 1993, describes the use of histidine buffers to chemically stabilize growth hormone formulations. Unfortunately, histidine is not a commercially viable buffer in many electrotransport drug formulations due to its instability in aqueous solution, thereby making the shelf-life of the drug formulation unacceptably short.

Controlling pH and assuring conductivity of electrotransport formulations is a dilemma that has not been solved to date. Control of pH in electrotransport systems is usually achieved by introduction of classic buffers such as TRIS, acetate or phosphate buffers in the formulation. This results in introduction of competing ions (i.e., ions having the same sign charge as the drug ions) into the drug formulation. In addition, in these formulations, donor reservoir pH drifting (i.e., during device operation) and reduced conductivity occurs during transport due to depletion of the charged species. This is of particular concern when the electrotransport delivery of therapeutically active polypeptide drugs is considered. Because these compounds are present at low concentration in the donor reservoir formulation, the detrimental effects caused by competing ions, i.e., decreasing conductivity of the formulation, decreasing transdermal drug flux, formulation pH drifting, and local skin irritation, are likely to be more severe. Recently, crosslinked ion exchange polymers have been used in an attempt to solve this deadlock. To date, their use has raised additional problems. In addition to regulatory concerns linked to the presence of small molecular weight degradants in these polymers, it is now evident that they do not provide adequate electrical conductivity and their usefulness in controlling pH is still subject to debate. What is still needed is a method which provides pH control and conductivity of the electrotransport drug formulation without introduction of competing ions and which is accomplished with the use of small molecular weight compounds that are easy to characterize.

Although histidine has been used to buffer protein formulations (WO 93/12812), the use of hisitidine to buffer electrotransport drug formulations is problematic due to the poor chemical stability of histidine in aqueous solutions. Water is by far the most preferred liquid solvent for electrotransport drug formulations due to its excellent biocompatability when in contact with skin. The aqueous stability of histidine is so poor that the formulations are not able to achieve the minimum stable shelf life required by drug regulatory agencies.

Thus, alternative methods for buffering aqueous electrotransport drug formulations, and in particular polypeptide drug or protein formulations, would be desirable.

### Disclosure of the Invention

The present invention provides a buffered aqueous formulation for transdermal electrotransport delivery exhibiting excellent stability characteristics. The reservoir formulation may be a donor reservoir formulation containing a drug or other therapeutic agent to be transdermally delivered. Alternatively, the reservoir formulation may be a counter reservoir formulation containing an electrolyte (e.g., saline). The formulation comprises an aqueous solution of the drug or electrolyte buffered with a dipeptide buffer. The dipeptide buffer comprises a polypeptidic chain of two to five amino acids, and has an isoelectric pH at which the dipeptide carries no net charge. The aqueous solution has a pH which is within about 1.0 pH unit of the isoelectric pH. Preferably, the dipeptide has at least two pKa's which are separated by no more than about 3.5 pH units. Most preferably, the isoelectric pH of the dipeptide is between about 3 and 10. The concentration of the dipeptide buffer in the solution is preferably at least about 10mM. The dipeptide buffer is preferably selected from the group consisting of Asp-Asp, Gly-Asp, Asp-His, Glu-His, His-Glu, His-Asp, Glu-Arg, Glu-Lys, Arg-Glu, Lys-Glu, Arg-Asp, Lys-Asp, His-Gly, His-Ala, His-Asn, His-Citruline, His-Gln, His-Hydroxyproline, His-Isoleucine, His-Leu, His-Met, His-Phe, His-Pro, His-Ser, His-Thr, His-Trp, His-Tyr, His-Val, Asn-His, Thr-His, Try-His, Gin-His, Phe-His, Ser-His, Citruline-His, Trp-His, Met-His, Val-His, His-His, Isoleucine-His, Hydroxyproline-His, Leu-His, Ala-His, Gly-His, Beta-Alanylhistidine, Pro-His, Carnosine, Anserine, Tyr-Arg, Hydroxylysine-His, His-Hydroxytlysine, Ornithine-His, His-Lys, His-Ornithine and Lys-His. A particularly preferred dipeptide buffer is Gly-His.

The present invention also provides a method of buffering an aqueous solution of a drug or an electrolyte used for transdermal electrotransport delivery. The method includes providing in the solution a pH buffering amount of a dipeptide comprising a polypeptidic chain of two to five amino acids, and having an isoelectric pH at which the dipeptide carries no net charge. The aqueous solution has a pH which is within about 1.0 pH unit of the isoelectric pH. Preferably, the dipeptide has at least two pKa's which are separated by no more than about 3.5 pH units. Most preferably, the isoelectric pH of the dipeptide is between about 3 and 10. The concentration of the dipeptide buffer in the solution is preferably at least about 10mM. The dipeptide buffer is preferably selected from the group consisting of Asp-Asp, Gly-Asp, Asp-His, Glu-His, His-Glu, His-Asp, Glu-Arg, Glu-Lys, Arg-Glu,, Lys-Glu, Arg-Asp, Lys-Asp, His-Gly, His-Ala, His-Asn, His-Citruline, His-Gln, His-Hydroxyproline, His-Isoleucine, His-Leu, His-Met, His-Phe, His-Pro, His-Ser, His-Thr, His-Trp, His-Tyr, His-Val, Asn-His, Thr-His, Try-His, Gin-His, Phe-His, Ser-His, Citruline-His, Trp-His, Met-His, Val-His, His-His, Isoleucine-His, Hydroxyproline-His, Leu-His, Ala-His, Gly-His, Beta-Alanylhistidine, Pro-His, Carnosine, Anserine, Tyr-Arg, Hydroxylysine-His, His-Hydroxytlysine, Ornithine-His, His-Lys, His-Ornithine and Lys-His. A particularly preferred dipeptide buffer is Gly-His.

### Brief Description of the Drawings

Figure 1 is a graph showing ionic charge versus pH for the dipeptide buffer Gly-His.
Figure 2 is a graph showing charged ion species distribution versus pH for the dipeptide buffer Gly-His.
Figure 3 is a graph showing ionic charge versus pH for two prior art buffers.
Figure 4 is a graph showing charged ion species distribution versus pH for phosphoric acid, a prior art buffer.
Figure 5 is a graph of charged ion species distribution versus pH for 3-[N-morpholino]propanesulphonic acid (MOPS), a prior art buffer.
Figure 6 is a graph of charged ion species distribution versus pH for the dipeptide buffer Glu-His.
Figure 7 is a graph of charged ion species distribution versus pH for the dipeptide buffer His-Glu.
Figure 8 is an exploded view of a representative electrotransport drug delivery device which can be used with the present invention.
Figure 9 is a graph of human growth hormone degradation versus time using a Gly-His dipeptide buffer.
Figure 10 is a graph of human growth hormone degradation versus time using His, a non-dipeptide buffer.
Figure 11 is a graph of transdermal flux of a model decapeptide at varying Gly-His concentrations.

### Modes for carrying out the Invention

The practice of the present invention will employ, unless otherwise indicated, conventional methods of protein chemistry, electrochemistry and biochemistry within the skill of the art. Such techniques are explained fully in the literature. See, e.g., T.E. Creighton, Proteins: Structures and Molecular Properties (W.H. Freeman and Company, 1993); A.L. Lehninger, Biochemistry (Worth Publishers, Inc., 1975); J.S. Newman, Electrochemical Systems (Prentice Hall, 1973); and A.J. Bard and L.R. Faulkner, Electrochemical Methods, Fundamentals and Applications (John Wiley & Sons, 1980).

It must be noted that, as used in this specification and the appended claims, the singular forms "a", "an" and "the" include plural referents unless the content clearly dictates otherwise. Thus, for example, reference to "a polypeptide" includes a mixture of two or more polypeptides, and the like.

The following amino acid abbreviations are used throughout the text:

| | |
|---|---|
| Alanine: Ala (A) | Arginine: Arg (R) |
| Asparagine: Asn (N) | Aspartic acid: Asp (D) |
| Cysteine: Cys (C) | Glutamine: Gln (Q) |
| Glutamic acid: Glu (E) | Glycine: Gly (G) |
| Histidine: His (H) | Isoleucine: lle (I) |
| Leucine: Leu (L) | Lysine: Lys (K) |
| Methionine: Met (M) | Phenylalanine: Phe (F) |
| Proline: Pro (P) | Serine: Ser (S) |
| Threonine: Thr (T) | Tryptophan: Trp (W) |
| Tyrosine: Tyr (Y) | Valine: Val (V) |

### I. Definitions

In describing the present invention, the following terms will be employed, and are intended to be defined as indicated below.

The term "dipeptide" denotes any polypeptidic chain of 2 to 5 amino acid residues. The term encompasses dipeptides, tripeptides, tetrapeptides, and pentapeptides, and particularly includes dipeptides and tripeptides which contain His, such as but not limited to, His-Gly, Gly-His, Ala-His, His-Ser and His-Ala.

The term "drug" and "therapeutic agent" are used interchangeably and are intended to have their broadest interpretation as any therapeutically active substance which is delivered to a living organism to produce a desired, usually beneficial, effect. In general, this includes therapeutic agents in all of the major therapeutic areas including, but not limited to, anti-infectives such as antibiotics and antiviral agents, analgesics including fentanyl, sufentanil, buprenorphine and analgesic combinations, anesthetics, anorexics, antiarthritics, antiasthmatic agents such as terbutaline, anticonvulsants, antidepressants, antidiabetic agents, antidiarrheals, antihistamines, anti-inflammatory agents, antimigraine preparations, antimotion sickness preparations such as scopolamine and ondansetron, antinauseants, antineoplastics, antiparkinsonism drugs, antipruritics, antipsychotics, antipyretics, antispasmodics, including gastrointestinal and urinary, anticholinergics, antiulceratives such as ranitidine, sympathomimetrics, xanthine derivatives, cardiovascular preparations including calcium channel blockers such as nifedipene, beta-blockers, beta-agonists such as dobutamine and ritodrine, antiarrythmics, antihypertensives such as atenolol, ACE inhibitors such as enalapril, benzodiazepine antagonists such as flumazenil, diuretics, vasodilators, including general, coronary, peripheral and cerebral, central nervous system stimulants, cough and cold preparations, decongestants, diagnostics, hormones such as parathyroid hormone, hypnotics, immunosuppressives, muscle relaxants, parasympatholytics, parasympathomimetrics, prostaglandins, proteins, peptides, psychostimulants, sedatives and tranquilizers.

The invention is also useful in the controlled delivery of polypeptide and protein drugs and other macromolecular drugs. These macromolecular substances typically have a molecular weight of at least about 300 daltons, and more typically a molecular weight in the range of about 300 to 40,000 daltons. Specific examples of peptides, and proteins and macromolecules in this size range include, without limitation, LHRH, LHRH analogs such as buserelin, gonadorelin, napharelin and leuprolide, GHRH, GHRF, insulin, insulotropin, heparin, calcitonin, octreotide, endorphin, TRH, NT-36 (chemical name: N=[[(s)-4-oxo-2-azetidinyl]carbonyl]-L-histidyl-L-prolinamide), liprecin, pituitary hormones (e.g., HGH, HMG, HCG, desmopressin acetate, etc.), follicle luteoids, αANF, growth factors such as growth factor releasing factor (GFRF), βMSH, somatostatin, atrial natriuretic peptide, bradykinin, somatotropin, platelet-derived growth factor, asparaginase, bleomycin sulfate, chymopapain, cholecystokinin, chorionic gonadotropin, corticotropin (ACTH), epidermal growth factor, erythropoietin, epoprostenol (platelet aggregation inhibitor), follicle stimulating hormone, glucagon, hirulog, and other analogs of hirudin, hyaluronidase, interferon, insulin-like growth factors, interleukin-1, interleukin-2, menotropins (urofollitropin (FSH) and LH), oxytocin, streptokinase, tissue plasminogen activator, urokinase, vasopressin, desmopressin, ACTH analogs, ANP, ANP clearance inhibitors, angiotensin II antagonists, antidiuretic hormone agonists, antidiuretic hormone antagonists, bradykinin antagonists, CD4, ceredase, CSF's, enkephalins, FAB fragments, IgE peptide suppressors, IGF-1, neuropeptide Y, neurotrophic factors, oligodeoxynucleotides and their analogues such as antisense RNA, antisense DNA and anti-gene nucleic acids, opiate peptides, colony stimulating factors, parathyroid hormone and agonists, parathyroid hormone antagonists, prostaglandin antagonists, pentigetide, protein C, protein S, ramoplanin, renin inhibitors, thymosin alpha-1, thrombolytics, TNF, vaccines, vasopressin antagonist analogs, alpha-1 anti-trypsin (recombinant), and TGF-beta. With electrotransport delivery devices, it has been recognized that the agents should generally be soluble in water. It is generally believed that the pathways for electrotransport drug delivery are hydrophilic pathways or pores such as those associated with hair follicles and sweat glands. The preferred form of an agent for electrotransport delivery is hydrophilic (e.g., water soluble salt form).

The term " transdermal delivery" refers to the delivery through a body surface (e.g., skin) of one or more pharmaceutically active agents to be available for either a local or systemic pharmacological effect. Penetration enhancers can be used to facilitate absorption through the skin. Such penetration enhancers include solvents such as water, alcohols including methanol, ethanol, 2-propanol, dodecanol, dodecanediol and the like, alkyl methyl sulfoxides, pyrrolidones, laurocapram, acetone, dimethylacetamide, dimethyl formamide, tetrahydrofurfuryl; surfactants including fatty acids/salts such as laurates; and chemicals such as urea, N,N-diethyl-m-toluamide, and the like.

The terms "electrotransport", "iontophoresis", and "iontophoretic" are used herein to refer to the delivery through a body surface (e.g., skin) of one or more pharmaceutically active agents by means of an applied electromotive force to an agent-containing reservoir. The agent may be delivered by electromigration, electroporation, electroosmosis or any combination thereof. Electroosmosis has also been referred to as electrohydrokinesis, electro-convection, and electrically induced osmosis. In general, electroosmosis of a species into a tissue results from the migration of solvent in which the species is contained, as a result of the application of electromotive force to the therapeutic species reservoir, i.e., solvent flow induced by electromigration of other ionic species. During the electrotransport process, certain modifications or alterations of the skin may occur such as the formation of transiently existing pores in the skin, also referred to as "electroporation". Any electrically assisted transport of species enhanced by modifications or alterations to the body surface (e.g., formation of pores in the skin) are also included in the term "electrotransport" as used herein. Thus, as used herein, the terms "electrotransport", "iontophoresis" and "iontophoretic" refer to (1) the delivery of charged agents by electromigration, (2) the delivery of uncharged agents by the process of electroosmosis, (3) the delivery of charged or uncharged agents by electroporation, (4) the delivery of charged agents by the combined processes of electromigration and electroosmosis, and/or (5) the delivery of a mixture of charged and uncharged agents by the combined processes of electromigration and electroosmosis.

Transdermal electrotransport flux can be assessed using a number of in vivo or in vitro methods, well known in the art. In vitro methods include clamping a piece of skin of an appropriate animal (e.g., human cadaver skin) between the donor and receptor compartments of an electrotransport flux cell, with the stratum comeum side of the skin piece facing the donor compartment. A liquid solution or gel containing the drug to be delivered is placed in contact with the stratum corneum, and electric current is applied to electrodes, one electrode in each compartment. The transdermal flux is calculated by sampling the amount of drug in the receptor compartment. Two successful models used to optimize transdermal electrotransport drug delivery are the isolated pig skin flap model of Riviere, Heit et al, J. Pharm. Sci. (1993) 82:240-243, and the use of isolated hairless skin from hairless rodents or guinea pigs. See, Hadzija et al., J. Pharm. Pharmacol. (1992) 44:387-390. See, also, Ogiso et al., Biol. Pharm. Bull. (1996) 19:1049-1054, for a description of a method for evaluating percutaneous absorption of insulin.

### II. Modes of Carrying Out the Invention

The present invention concerns the use of dipeptides to buffer transdermal electrotransport reservoir formulations, particularly drug-containing donor reservoir formulations and more particularly donor reservoir formulations used for electrotransport delivery of a polypeptide or protein drug. The method therefore permits increased efficiency of the transdermal delivery of a large number of substances, and allows for the transdermal delivery of molecules that would not otherwise be amenable to such delivery.

In performing electrotransport experiments in animals, it has surprisingly been discovered that some buffers are better suited for pH control. In particular, dipeptide buffers such as Gly-His and His-Glu at their pl are capable of assuring pH control of electrotransport formulations for several hours. Dipeptide buffers for use in the present invention include dipeptides, tripeptides, tetrapeptides, and pentapeptides which contain His, such as His-Gly, Gly-His, Ala-His, L-carnosine (also known as L-Ala-His), His-Ser, His-Ala, Gly-Gly-His (pl = 7.5), His-Gly-Gly (pl = 6.9), Gly-Gly-Gly-His (pl = 7.5), His-Gly-Gly-Gly (pi = 6.9), Gly-Gly-Gly-Gly-His (pl = 7.55), and His-Gly-Gly-Gly-Gly (pl = 6.0).

The dipeptide should have at least two pKa's separated by no more than about 3.5 pH units. Beyond this range, pH control will be poor and conductivity of the solution will be minimal. The pl range of the dipeptide should be between 3 and 10 and the pH of the formulation should be no more than about 1 pH unit away from the isoelectric pH (i.e., the pl) of the dipeptide. Generally, the formulation pH will be from about pH 3 to about pH 9.5. However, the preferred formulation pH will depend on the particular drug and dipeptide buffer used in the formulation. Beyond these pH limits (i.e., less than pH 3 and greater than pH 10), the formulation is likely to be irritating or will result in unacceptable skin resistance. In addition, if the formulation pH is more than 1 pH unit away from the pl of the dipeptide buffer, the effects described above will be inefficient as the dipeptide will start behaving like a conventional buffer (high transport efficiency of charged species and pH drifting). When the dipeptide is used in a solution having a pH at or close to the pl of the dipeptide (i.e., pl ± 1.0 pH unit), minimum competition with the drug ions (i.e., for electrotransport into the patient) will occur because the buffer is at or close to electrical (i.e., ionic) neutrality and therefore it can be used with good results (i.e., little or no ionic competition with the drug ions) in either the anode or the cathode reservoir formulations. If for technical reasons it is decided to use the dipeptide at a pH between 0.5 to 1.0 pH unit away from the pl, the use of the buffer at a pH slightly higher than its pl is preferred in the cathodic formulation in order to minimize ionic competition with the drug being delivered. Conversely, and for the same reason, the use of the dipeptide buffer at a pH slightly below (i.e., between 0.5 to 1.0 pH unit below) its pl is preferred in the anodic formulation. In the counter reservoir formulation (i.e., the non-drug containing reservoir) this preference is not as important as there is no concern over the buffer ions competing with drug ions for delivery into the patient from the counter reservoir. The dipeptide buffer will generally be present in the formulation at a concentration of from about 10 mM to 1 M, more preferably from about 10 mM to about 250 mM, and most preferably from about 25 mM to about 250 mM.

Table 1 lists conductivities and solubilities of selected dipeptides useful in the present invention, at their pl.

**TABLE 1**

| **Dipeptide** | **pl** | **Conductivity at 10**^{**-2**} **Molar (µS*/cm)** | **Solubility (Moles/l)** |
|---|---|---|---|
| His-Glu | 5.20 | 40 | 0.40 |
| His-Asp | 5.22 | 28 | 0.05 |
| Glu-Lys | 6.00 | 6 | 1.00 |
| Lys-Glu | 6.06 | 8 | 0.50 |
| Lys-Asp | 6.08 | 6 | 1.00 |
| His-Gly | 6.90 | 40 | 1.00 |
| His-Ala | 6.95 | 60 | 0.50 |
| Val-His | 7.38 | 94 | 0.20 |
| Gly-His | 7.55 | 52 | 1.00 |

| | | | |
|---|---|---|---|
| *micro Siemens | | | |

The dipeptide buffer preferably includes at least one amino acid selected from His, Asp, Glu, Lys, Tyr, Arg and Cys; more preferably includes at least one amino acid selected from His, Asp, Glu, and Lys; and most preferably includes at least one amino acid selected from His and derivatives thereof (e.g., methyl-His).

This invention can be practiced in many different ways. In its simplest form, the dipeptide provides pH control to the formulation containing no drug contained in the counter electrode (cathode or anode) reservoir of the electrotransport system. The dipeptide may also be incorporated in the donor (i.e., drug-containing) reservoir formulation (cathodic or anodic).

The buffering of the anodic and/or cathodic reservoirs of a transdermal electrotransport drug delivery device is particularly important because these reservoirs must contain a liquid solution of a drug or other electrolyte. The liquid solvent used for the drug/electrolyte solutions is usually water due to water's excellent biocompatibility. During operation of an electrotransport device, an oxidation reaction takes place at the interface between the anodic electrode and the solution contained in the anodic reservoir. Similarly, an electrochemical reduction reaction takes place at the interface between the cathodic electrode and the solution in the cathodic reservoir. When the electrodes are composed of electrochemically nonreactive materials, such as platinum or stainless steel, the water tends to be the primary species which is either oxidized or reduced, thereby causing a pH drop in the anodic reservoir and a pH rise in the cathodic reservoir. See for example, Phipps, et al., U.S. Patent 4,744,787; Phipps, et al., U.S. Patent 4,747,819; and Petelenz, et al., U.S. Patent 4,752,285. Although the use of electrochemically reactive electrode materials, such as a silver anode and/or a silver chloride cathode substantially reduces the oxidation and reduction of water in electrotransport reservoirs as taught in the above-identified Phipps, et al. and Petelenz, et al. patents, there is still some tendency for the water in these reservoirs to be oxidized or reduced during operation of the device, leading to undesirable pH changes. Thus, while the dipeptide buffers of the present invention have particular utility in those electrotransport devices utilizing electrodes composed of materials which are electrochemically nonreactive, the buffers of the present invention can still find utility even in those electrotransport devices utilizing electrodes composed of electrochemically reactive materials.

Many dipeptides present adequate characteristics for use in electrotransport formulation. Table 2 includes a non-exhaustive list of the dipeptide buffers ranked by increasing pl. Dipeptides having up to five amino acids and containing the amino acids histidine, lysine, aspartic acid or glutamic acid in combination or with other amino acids are particularly useful to this invention.

**TABLE 2**

| **Dipeptide** | **pka A** | **pka A** | **pka A** | **pka B** | **pka B** | **pka B** | **pl** | **% salt** |
|---|---|---|---|---|---|---|---|---|
| Asp-Asp | 2.70 | 3.40 | 4.70 | 8.26 | | | 3.05 | 43 |
| Gly-Asp | 2.81 | 4.45 | | 8.60 | | | 3.60 | 23 |
| Asp-His | 2.45 | 3.02 | | 6.81 | 7.98 | | 4.90 | 3 |
| Glu-His | 2.45 | 3.45 | | 6.81 | 8.20 | | 5.20 | 5 |
| His-Glu | 2.30 | 4.19 | | 6.32 | 8.07 | | 5.20 | 15 |
| His-Asp | 2.28 | 3.99 | | 6.45 | 8.19 | | 5.22 | 11 |
| Glu-Arg | 2.66 | 4.01 | | 7.94 | 12.50 | | 6.00 | 2 |
| Glu-Lys | 2.85 | 4.01 | | 7.94 | 11.07 | | 6.00 | 2 |
| Arg-Glu | 2.74 | 4.18 | | 7.92 | 12.50 | | 6.06 | 3 |
| Lys-Glu | 2.74 | 4.18 | | 7.92 | 11.12 | | 6.06 | 3 |
| Arg-Asp | 2.64 | 4.10 | | 8.05 | 12.50 | | 6.08 | 2 |
| Lys-Asp | 2.64 | 4.10 | | 8.05 | 11.20 | | 6.08 | 2 |
| His-Gly | 2.41 | | | 5.90 | 7.91 | | 6.90 | 16 |
| His-Ala | 2.48 | | | 6.10 | 7.80 | | 6.95 | 22 |
| His-Asn | 2.62 | | | 6.10 | 7.80 | | 6.95 | 22 |
| His-Citrulline | 3.05 | | | 6.10 | 7.80 | | 6.95 | 22 |
| His-Gln | 2.93 | | | 6.10 | 7.80 | | 6.95 | 22 |
| His-Hydroxyproline | 2.42 | | | 6.10 | 7.80 | | 6.95 | 22 |
| His-isoleucine | 3.13 | | | 6.10 | 7.80 | | 6.95 | 22 |
| His-Leu | 3.10 | | | 6.10 | 7.80 | | 6.95 | 22 |
| His-Met | 2.89 | | | 6.10 | 7.80 | | 6.95 | 22 |
| His-Phe | 2.88 | | | 6.10 | 7.80 | | 6.95 | 22 |
| His-Pro | 2.62 | | | 6.10 | 7.80 | | 6.95 | 22 |
| His-Ser | 2.65 | | | 6.10 | 7.80 | | 6.95 | 22 |
| His-Thr | 2.98 | | | 6.10 | 7.80 | | 6.95 | 22 |
| His-Trp | 3.07 | | | 6.10 | 7.80 | | 6.95 | 22 |
| His-Tyr | 2.13 | 9.97 | | 6.10 | 7.80 | | 6.95 | 22 |
| His-Val | 3.18 | | | 6.10 | 7.80 | | 6.95 | 22 |
| Asn-His | 2.42 | | | 6.71 | 7.30 | | 7.00 | 44 |
| Thr-His | 2.42 | | | 6.71 | 7.60 | | 7.15 | 39 |
| Tyr-His | 2.42 | 9.90 | | 6.71 | 7.60 | | 7.15 | 39 |
| Gln-His | 2.42 | | | 6.71 | 7.70 | | 7.20 | 36 |
| Phe-His | 2.42 | | | 6.71 | 7.70 | | 7.20 | 36 |
| Ser-His | 2.42 | | | 6.71 | 7.70 | | 7.20 | 36 |
| Citrulline-His | 2.42 | | | 6.71 | 7.90 | | 7.30 | 32 |
| Trp-His | 2.42 | | | 6.71 | 7.90 | | 7.30 | 32 |
| Met-His | 2.42 | | | 6.71 | 7.97 | | 7.35 | 30 |
| Val-His | 3.09 | | | 6.83 | 7.94 | | 7.38 | 34 |
| His-His | 2.25 | | | 5.40 | 6.80 | 7.95 | 7.40 | 32 |
| Isoleucine-His | 2.42 | | | 6.71 | 8.20 | | 7.44 | 25 |
| Hydroxyproline-His | 2.42 | | | 6.71 | 8.23 | | 7.45 | 25 |
| Leu-His | 2.42 | | | 6.71 | 8.25 | | 7.50 | 24 |
| Ala-His | 2.42 | | | 6.71 | 8.37 | | 7.55 | 22 |
| Gly-His | 2.42 | | | 6.71 | 8.39 | | 7.55 | 22 |
| Beta-Alanylhistidine | 2.60 | | | 6.70 | 8.70 | | 7.70 | 16 |
| Pro-His | 2.42 | | | 6.71 | 9.10 | | 7.90 | 11 |
| Carnosine | 2.64 | | | 6.83 | 9.51 | | 8.17 | 8 |
| Anserine | 2.64 | | | 7.04 | 9.49 | | 8.27 | 10 |
| Tyr-Arg | 2.64 | 9.36 | | 7.39 | 11.62 | | 8.40 | 17 |
| Hydroxylysine-His | 2.42 | | | 6.71 | 7.40 | 9.70 | 8.60 | 13 |
| His-Hydroxylysine | 3.05 | | | 6.10 | 7.80 | 9.70 | 8.75 | 17 |
| Ornithine-His | 2.42 | | | 6.71 | 7.30 | 11.00 | 9.20 | 3 |
| His-Lys | 3.05 | | | 6.10 | 7.80 | 11.00 | 9.40 | 5 |
| His-Ornithine | 2.82 | | | 6.10 | 7.80 | 11.00 | 9.40 | 5 |
| Lys-His | 2.42 | | | 6.71 | 8.00 | 11.00 | 9.50 | 5 |
| pKa A = acidic pKa | | | | | | | | |
| pKa B = basic pKa | | | | | | | | |
| % salt = fraction of the dipeptide that is ionized and carries a net positive and/or negative charge, but not including the ionized species carrying a net neutral charge, in an aqueous solution having a pH equal to the pl | | | | | | | | |

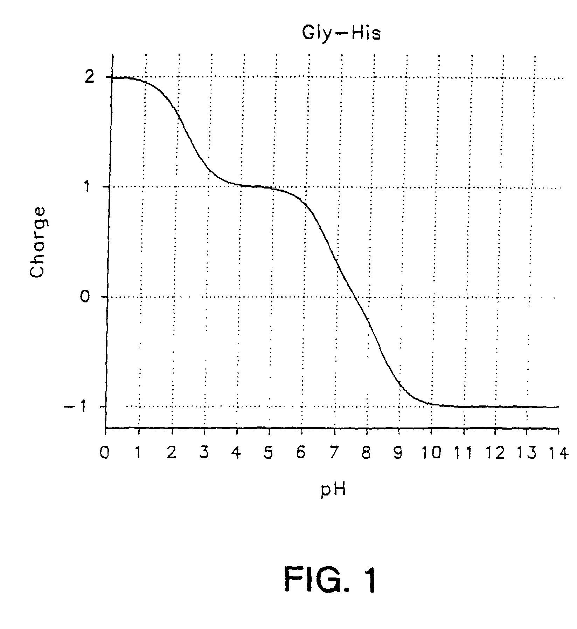

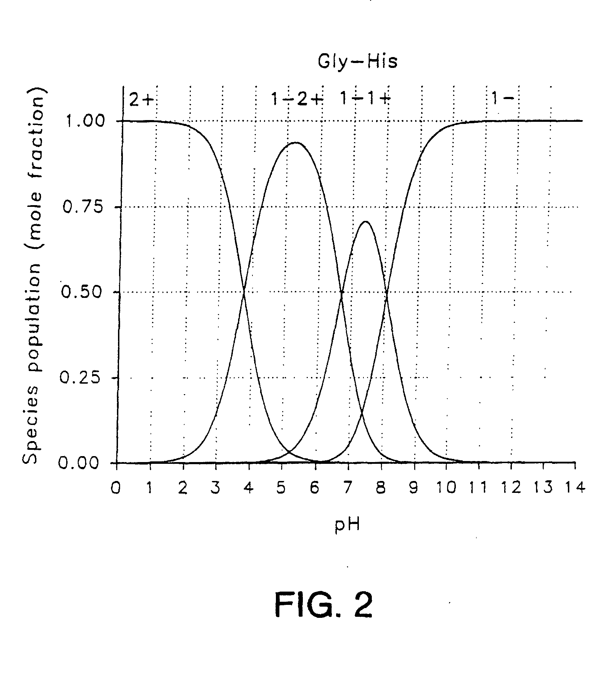

The pH buffering capacity of the dipeptide buffers of the present invention can be explained by using Gly-His at pH 7.5 as an example (the pi of Gly-His is 7.55). At this pH, the net charge of the molecule is essentially zero (see Figure 1). At the pl, three species coexist. The bulk of the molecule (70%) consists of the neutral species which bears two internal charges, one positive and one negative resulting in a net charge of zero. The remaining (30%) consists of the salt form of the positively charged species (1-2+, net charge = +1) and the negatively charged species (-1); see Figure 2). The existence of this salt can be demonstrated by measuring the conductivity of the solution of a Gly-His solution at its pl (Table 1). Although there are small percentages of species presenting a net positive or negative charge in solution, there is minimal ionic transport of these charged species due to charge equilibrium between the three species (i.e., a positive charge migrating in the electric field will revert almost instantly to its neutral form and lose momentum or to its negative form and migrate backward). Due to the same principle of charge equilibrium, any depletion of the charged molecules will be compensated immediately by dissociation of the neutral form to its charged species thereby providing a reservoir insuring long term pH stability. In addition, if loss of the molecule occurs by electroosmosis of the neutral species, this will not result in any pH changes.

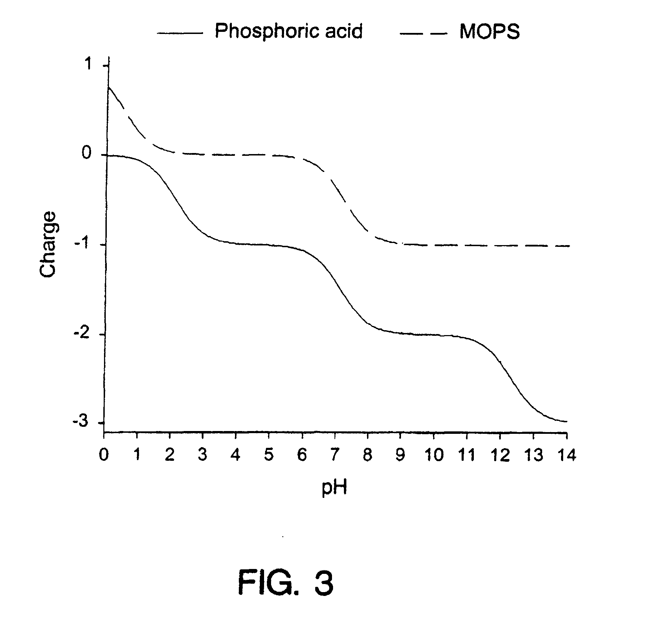

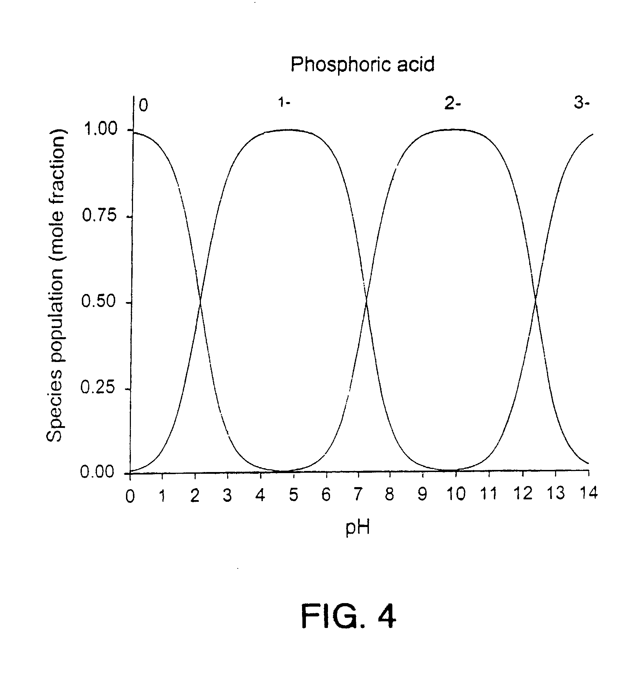

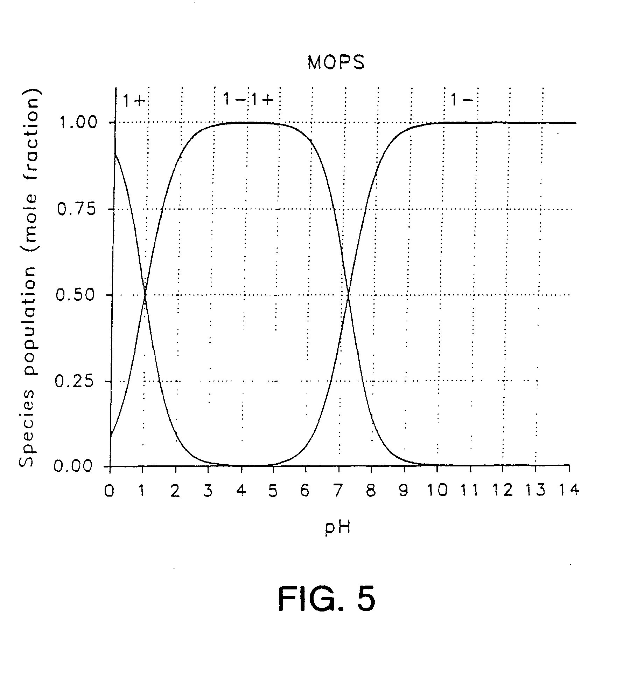

The pH buffering capacity of dipeptides at or near their isoelectric pH contrasts sharply with the lack of pH stability observed with conventional buffers such as phosphate or 3-[N-morpholino] propane sulfonic acid (MOPS) at the same or higher ionic strength as shown in Table 3. The decrease of pH observed in the presence of phosphate (a triacid: pKa's of 2.12, 7.2 and 12.32) and MOPS (a zwitterion: pKa's of <1 and 7.2) can be explained by the fact that these buffers are used at a pH close to their pKa. The net charge of phosphoric acid and MOPS at pH 7 is respectively -1/5 and -0.5 (see Figure 3). For phosphoric acid, half of the molecules have a -2 valence and the other half have a -1 valence (see Figure 4). For MOPS, half of the molecules have a -1 valence and the other half are the neutral form of the molecule which bears two internal charges, one positive and one negative (see Figure 5). In an electric field these negative species move in the opposite direction of their positive associated counterions. This migration will result in depletion of the charged species and accumulation of the neutral species in the reservoir resulting in a pH drop. At its pl, MOPS (pl = 4) does not present any buffering capacity and MOPS solutions at this pH are nonconductive.

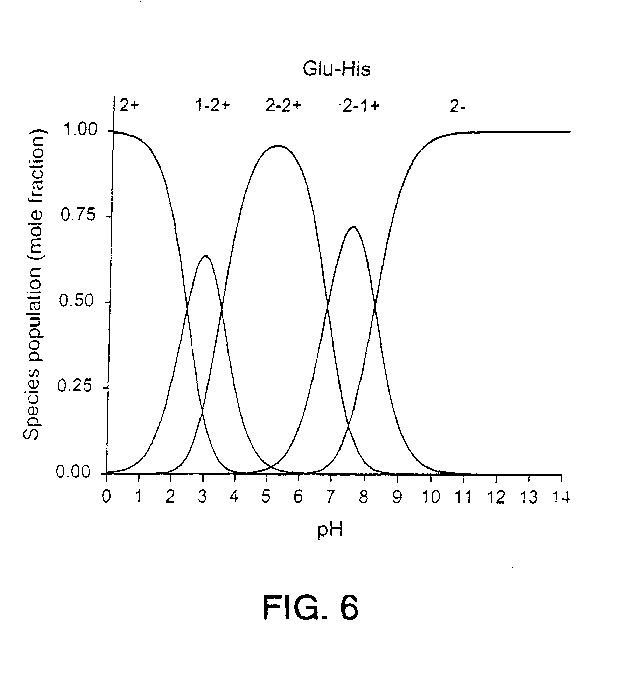

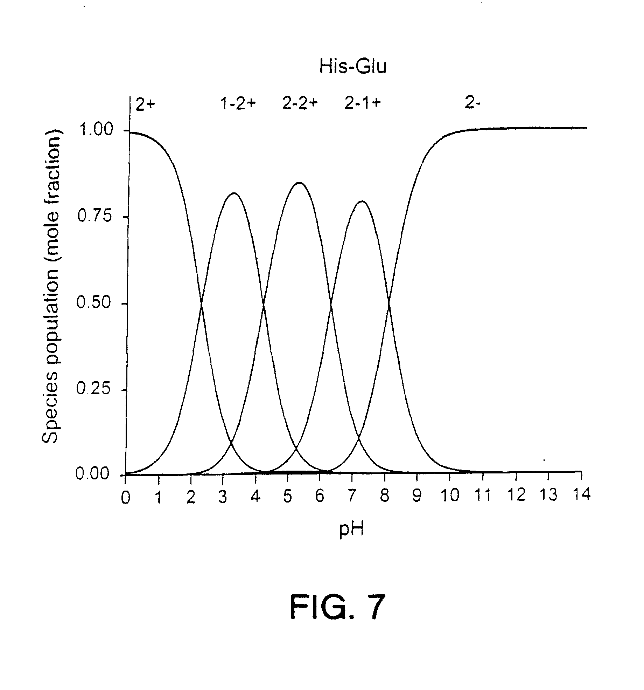

Figures 6 and 7 present examples of the charge distribution for two dipeptides (Glu-His and His-Glu) both having a pl of 5.2. At the pl, the species presenting a net charge (which assure electrical conductivity) represent respectively about 5% and 15% of the molecules. Choice of the dipeptide buffer will be on a case-by-case basis depending on the drug compound and the desired level of pH control and conductivity of the formulation. For example with a non-peptidic drug such as fentanyl, conductivity of the buffer and tight pH control is not essential because the drug itself is present at high concentration which provides adequate conductivity and because the charge of the drug is constant in the pH range zero to seven. For this drug, the buffer Glu-His is a perfect choice. The pl of this buffer is 5.2 (this pH assures solubility of the drug) and the conductivity of the buffer is minimal. If more pH control and more conductivity is required, as with most polypeptide and protein drugs such as goserelin, the buffer His-Glu is a judicious choice. The pl of this buffer is 5.2 (this pH assures that the goserelin has optimal charge) and about 15% of the buffer is charged at its pl assuring good conductivity of the formulation.

When used to buffer electrotransport donor (i.e., drug-containing) reservoir formulations, the present invention is useful for any number of categories of therapeutic agents (i.e., drugs) and the invention is not limited thereby. The invention has particular utility in buffering aqueous polypeptide and protein drug formulations because these drugs are typically present at low concentration in the donor reservoir formulation, the detrimental effects caused by competing ions, i.e., decreasing conductivity of the formulation, decreasing transdermal drug flux, formulation pH drifting, and local skin irritation, are likely to be more severe. Such protein and polypeptide drugs include those derived from eucaryotic, procaryotic and viral sources, as well as synthetic polypeptide drugs. Such polypeptide drugs include without limitation, polypeptide drugs which are antibiotics and antiviral agents, antineoplastics, immunomodulators, polypeptide hormones such as insulin, proinsulin, growth hormone, GHRH, LHRH, EGF, Somatostatin, SNX-111, BNP, insulinotropin, ANP, and glycoprotein hormones such as, FSH, LH, PTH and hCG.

Examples of protein drugs for use with the present methods include any commercially available insulins, such as, for example, recombinant human insulin from Sigma, St. Louis, MO, formulated as neutral solutions or suspensions of zinc insulin. Such preparations of insulin contain a minimum of two zinc ions bound per hexamer and have an insulin concentration from about 0.2 to about 3.0 mM (1 mg mL⁻¹ to 18 mg mL⁻¹). However, insulin preparations including higher concentrations of insulin, up to about 17 mM insulin will also find use herein.

The drug and dipeptide buffer are present in an aqueous solution since water is by far the most preferred liquid solvent for transdermal electrotransport drug delivery due to its excellent biocompatibility. In addition to water, other pharmaceutically acceptable excipients such as dextrose, glycerol, ethanol, and the like may also be present. If desired, the pharmaceutical composition to be administered may also contain minor amounts of nontoxic auxiliary substances such as wetting or emulsifying agents, preservatives, ion-binding agents and the like, for example, sodium acetate, sorbitan monolaurate, triethanolamine sodium acetate, triethanolamine oleate, etc. The choice of an appropriate excipient and additives is determined largely by the drug being delivered. For a discussion of drug formulations, see, e.g., Remington: The Science and Practice of Pharmacy, Mack Publishing Company, Easton, Pennsylvania, 19th edition, 1995. For protein drug formulations, such excipients include, without limitation, preservatives such as methylparaben and phenol (m-cresol); isotonic agents such as glycerol or salts, including but not limited to NaCI (generally at a concentration of about 1 to about 100 mM NaCl); and the like. For a discussion of insulin formulations, see, e.g., Brange, J., Stability of Insulin (Kluwer Academic Publishers); Brange, J. Galenics of Insulin, The Physico-chemical and Pharmaceutical Aspects of Insulin and Insulin Preparations (Springer-Verlag); and Remington: The Science and Practice of Pharmacy, Mack Publishing Company, Easton, Pennsylvania, 19th edition, 1995.

Once the desired drug formulation with the dipeptide buffer is prepared, it can be used with any of several transdermal electrotransport drug delivery systems and use is not limited to any one particular electrotransport system. Examples of electrotransport drug delivery systems are described in, e.g., U.S. Patents 5,312,326 to Myers et al., 5,080,646 to Theeuwes et al., 5,387,189 to Gyory et al., and 5,169,383 to Gyory et al..

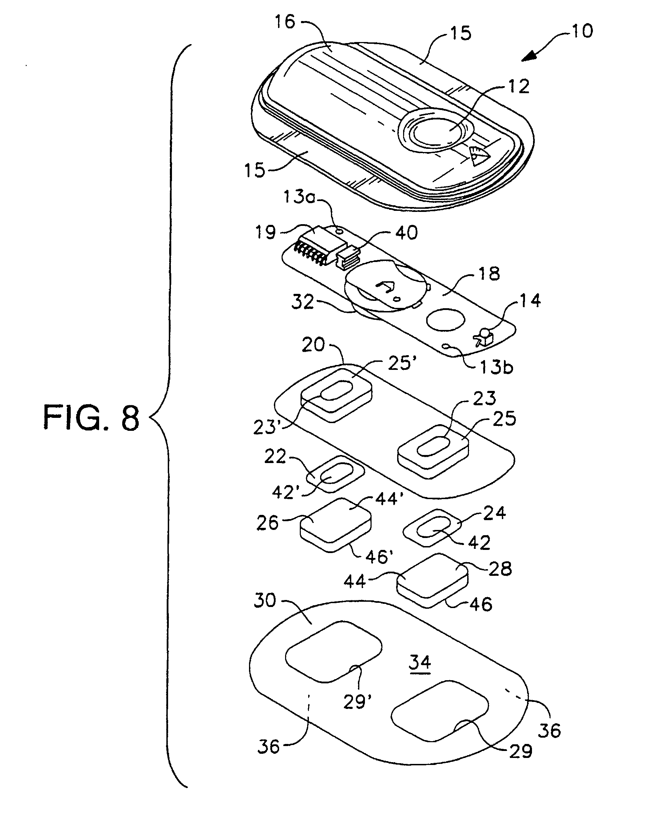

Figure 8 illustrates a representative electrotransport delivery device that may be used in conjunction with the present method. Device 10 comprises an upper housing 16, a circuit board assembly 18, a lower housing 20, anode electrode 22, cathode electrode 24, anode reservoir 26, cathode reservoir 28 and skin-compatible adhesive 30. When the drug to be delivered is cationic, anodic reservoir 26 will be the donor reservoir and cathodic reservoir 28 will be the counter reservoir. Conversely, when the drug to be delivered is anionic, cathodic reservoir 28 will be the donor reservoir and anodic reservoir 26 will be the counter reservoir.

Upper housing 16 has lateral wings 15 which assist in holding device 10 on a patient's skin. Upper housing 16 is preferably composed of an injection moldable elastomer (e.g., ethylene vinyl acetate). Printed circuit board assembly 18 comprises an integrated circuit 19 coupled to discrete components 40 and battery 32. Circuit board assembly 18 is attached to housing 16 by posts (not shown in Figure 2) passing through openings 13a and 13b, the ends of the posts being heated/melted in order to heat stake the circuit board assembly 18 to the housing 16. Lower housing 20 is attached to the upper housing 16 by means of adhesive 30, the upper surface 34 of adhesive 30 being adhered to both lower housing 20 and upper housing 16 including the bottom surfaces of wings 15.

Shown (partially) on the underside of circuit board assembly 18 is a button cell battery 32. Other types of batteries may also be employed to power device 10.

The device 10 is generally comprised of battery 32, electronic circuitry 19,40, electrodes 22,24, and drug/chemical reservoirs 26,28, all of which are integrated into a self-contained unit. The outputs (not shown in Figure 2) of the circuit board assembly 18 make electrical contact with the electrodes 24 and 22 through openings 23,23' in the depressions 25,25' formed in lower housing 20, by means of electrically conductive adhesive strips 42,42'. Electrodes 22 and 24, in turn, are in direct mechanical and electrical contact with the top sides 44',44 of drug reservoirs 26 and 28. The bottom sides 46',46 of drug reservoirs 26,28 contact the patient's skin through the openings 29',29 in adhesive 30.

Device 10 optionally has a feature which allows the patient to self-administer a dose of drug by electrotransport. Upon depression of push button switch 12, the electronic circuitry on circuit board assembly 18 delivers a predetermined DC current to the electrodes/reservoirs 22,26 and 24,28 for a delivery interval of predetermined length. The push button switch 12 is conveniently located on the top side of device 10 and is easily actuated through clothing. A double press of the push button switch 12 within a short time period, e.g., three seconds, is preferably used to activate the device for delivery of drug, thereby minimizing the likelihood of inadvertent actuation of the device 10. Preferably, the device transmits to the user a visual and/or audible confirmation of the onset of the drug delivery interval by means of LED 14 becoming lit and/or an audible sound signal from, e.g., a "beeper". Drug is delivered through the patient's skin by electrotransport, e.g., on the arm, over the predetermined delivery interval.

Anodic electrode 22 is preferably comprised of silver and cathodic electrode 24 is preferably comprised of silver chloride. Both reservoirs 26 and 28 are preferably comprised of polymer hydrogel materials. Electrodes 22,24 and reservoirs 26,28 are retained within the depressions 25',25 in lower housing 20.

The push button switch 12, the electronic circuitry on circuit board assembly 18 and the battery 32 are adhesively "sealed" between upper housing 16 and lower housing 20. Upper housing 16 is preferably composed of rubber or other elastomeric material. Lower housing 20 is preferably composed of a plastic or elastomeric sheet material (e.g., polyethylene) which can be easily molded to form depressions 25,25' and cut to form openings 23,23'. The assembled device 10 is preferably water resistant (i.e., splash proof) and is most preferably waterproof. The system has a low profile that easily conforms to the body, thereby allowing freedom of movement at, and around, the wearing site. The reservoirs 26 and 28 are located on the skin-contacting side of the device 10 and are sufficiently separated to prevent accidental electrical shorting during normal handling and use.

The device 10 adheres to the patient's body surface (e.g., skin) by means of a peripheral adhesive 30 which has upper side 34 and body-contacting side 36. The adhesive side 36 has adhesive properties which assures that the device 10 remains in place on the body during normal user activity, and yet permits reasonable removal after the predetermined (e.g., 24-hour) wear period. Upper adhesive side 34 adheres to lower housing 20 and retains lower housing 20 attached to upper housing 16.

The reservoirs 26 and 28 generally comprise a gel matrix, with the drug solution uniformly dispersed in at least one of the reservoirs 26 and 28. Drug concentrations in the range of approximately 1 x 10⁻⁴ M to 1.0 M or more can be used, with drug concentrations in the lower portion of the range being preferred. Suitable polymers for the gel matrix may comprise essentially any nonionic synthetic and/or naturally occurring polymeric materials. A polar nature is preferred when the active agent is polar and/or capable of ionization, so as to enhance agent solubility. Optionally, the gel matrix will be water swellable. Examples of suitable synthetic polymers include, but are not limited to, poly(acrylamide), poly(2-hydroxyethyl acrylate), poly(2-hydroxypropyl acrylate), poly(N-vinyl-2-pyrrolidone), poly(n-methylol acrylamide), poly(diacetone acrylamide), poly(2-hydroxylethyl methacrylate), poly(vinyl alcohol) and poly(allyl alcohol). Hydroxyl functional condensation polymers (i.e., polyesters, polycarbonates, polyurethanes) are also examples of suitable polar synthetic polymers. Polar naturally occurring polymers (or derivatives thereof) suitable for use as the gel matrix are exemplified by cellulose ethers, methyl cellulose ethers, cellulose and hydroxylated cellulose, methyl cellulose and hydroxylated methyl cellulose, gums such as guar, locust, karaya, xanthan, gelatin, and derivatives thereof. Ionic polymers can also be used for the matrix provided that the available counterions are either drug ions or other ions that are oppositely charged relative to the active agent.

Thus, the drug/dipeptide formulations of the present invention will be incorporated into the drug reservoir, e.g., a gel matrix as just described, and administered to a patient using an electrotransport drug delivery system, as exemplified hereinabove. Incorporation of the drug solution can be done any number of ways, i.e., by imbibing the solution into the reservoir matrix, by admixing the drug solution with the matrix material prior to hydrogel formation, or the like.

Optionally, the outermost layer of the skin may be punctured with a microblade array before electrotransport delivery therethrough. The mechanical cutting/puncturing of the stratum corneum is beneficial when transdermally delivering high molecular weight drugs such as polypeptides and proteins. Examples of microblade arrays, for either skin pretreatment or as an integrated feature of a transdermal electrotransport drug delivery device, are disclosed in Lee et al. U.S. Patent 5,250,023; Cormier et al. WO 97/48440; and Theeuwes et al. WO 98/28037.

While the invention has been described in conjunction with the preferred specific embodiments thereof, it is to be understood that the foregoing description as well as the examples which follow are intended to illustrate and not limit the scope of the invention. Other aspects, advantages and modifications within the scope of the invention will be apparent to those skilled in the art to which the invention pertains.

### Example 1

A sufficient quantity of His-Gly from BACHEM Bioscience was added to distilled water to make a 12.5 mM buffer solution having a pH of 6.75. A human growth hormone (hGH) formulation obtained from BresaGen contained growth hormone, mannitol and glycine in the following proportions: 1:5:1 (w/w). The original hGH formulation was subjected to purification (diafiltration against 12.5 mM His-Gly buffer to remove the mannitol and glycine) and the hGH concentration was adjusted to about 20 mg/ml via ultrafiltration.

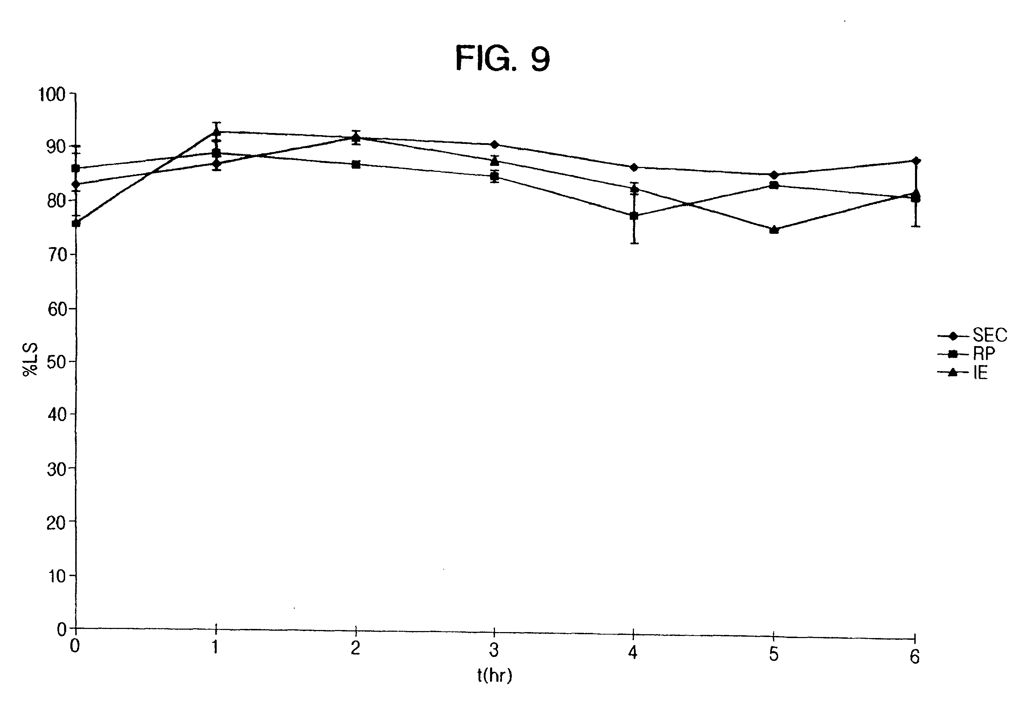

Aliquots of 250 µl of the resulting hGH stock solution were placed into Eppendorf tubes, each containing 5 mg (2%) of hydroxyethyl cellulose (HEC) as a gelling agent and the samples were carefully mixed. After gelation, the samples were tested for stability at body temperature. The samples were warmed to 32 °C (ie, skin temperature) and assayed at 0, 1, 2, 3, 4, 5, and 6 hours to determine the percent of hGH remaining intact in the gel. hGH from the gels was extracted by dissolving the gel in 25 ml of His-Gly buffer. All hGH samples were analyzed by reverse-phase high performance liquid chromatography (RP), size-exclusion high performance liquid chromatography (SEC), and ion-exchange high performance liquid chromatography (IE) to determine percentage of intact hGH remaining (%LS in Figure 9). The percent of hGH remaining was calculated by measuring the concentration of the hGH (as determined using one of three high performance liquid chromatography methods) and dividing that by the initial hGH concentration. The results of the His-Gly buffered hGH stability tests are shown in Figure 9.

When analyzed by these methods, no significant loss of protein through degradation was observed in the hGH gel formulations stored at 32 °C. No extra degradation products were discovered.

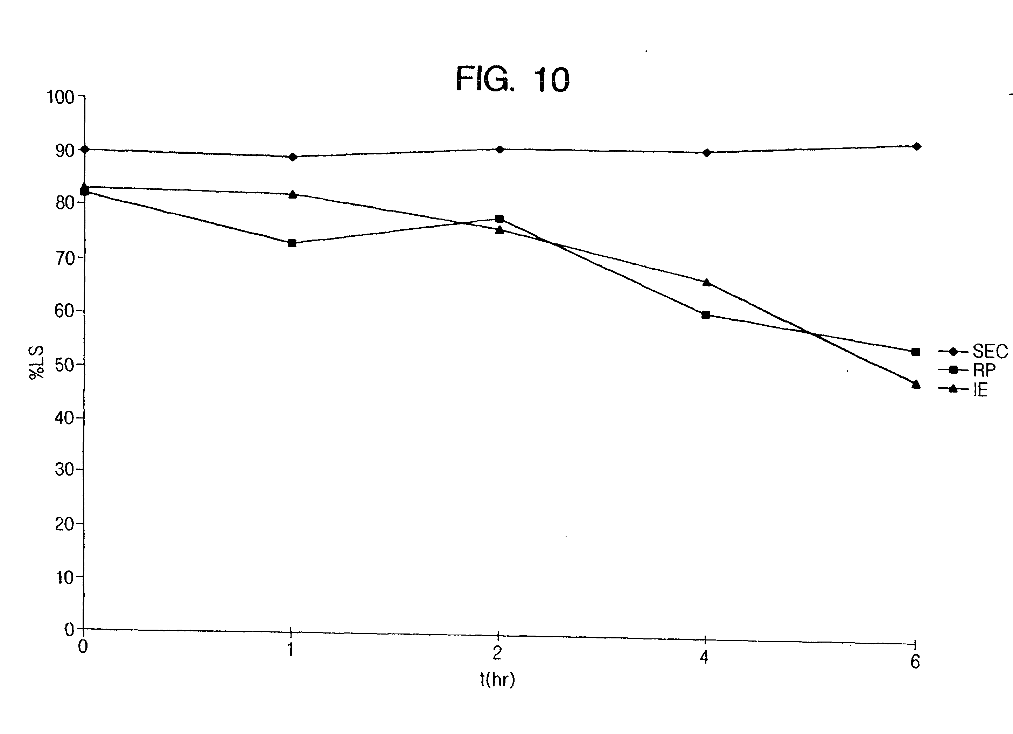

As a comparison, hGH stability studies were run under identical conditions to those described above, except histidine buffer was substituted for His-Gly. The results of the histidine stability tests are shown in Figure 10. Figure 10 shows that after only 6 hours, approximately 50% of the His buffered hGH remained intact, whereas about 80% of the hGH remained intact using the His-Gly buffer. As is clearly shown by comparing Figures 9 and 10, substitution of histidine buffer with His-Gly buffer considerably improved human growth hormone formulation stability.

### EXAMPLE 2

In vivo iontophoresis experiments were performed using custom built electrotransport systems. The anodic compartment comprised a skin-contacting gel containing the aqueous solution of the buffering agent at the indicated concentration and 3% of the gelling agent hydroxyethyl cellulose (HEC). This formulation was separated from the anode electrode by a Sybron ion exchange membrane. A gel containing 0.15M sodium chloride (which acted as the chloride source) was placed between the anode and the ionic exchange membrane. Alternatively, the anodic compartment comprised a skin-contacting gel containing the aqueous solution of the buffering agent at the indicated concentration and 3% HEC as well as 10% of the chloride source cholestyramine. The cathode-compartment comprised a skin-contacting gel containing the aqueous solution of the buffering agent at the indicated concentration and 3% HEC. This formulation was separated from the cathode electrode by a Nafion ion exchange membrane. A gel containing 0.15M sodium chloride was placed between the cathode and the ionic exchange membrane.

The systems had a silver foil anode and a silver chloride cathode. The reservoir gel (i.e., both the anodic and cathodic skin-contacting gels) sizes were each approximately 350 µL and had a skin contacting surface area of about 2 cm². The electrodes were connected to a DC power source which supplied a constant level of electric current of 0.1 mA/cm².

Experiments were performed in vivo in hairless guinea pigs. Three animals were used for each condition studied. The electrotransport systems were applied to and removed from the flank of the animals. The two reservoirs were generally spaced about 5 cm apart. The application site was wiped with water prior to system application. pH of the gels was measured prior to application and after removal of the systems using a HORIBA compact pH meter (Cardy).

Table 3 shows that Gly-His and His-Glu at their pl were capable of assuring pH control of an anodic electrotransport formulation for several hours. This contrasts with the lack of stability observed with buffers such as phosphate or MOPS at the same or higher ionic strength.

Table 4 shows that Gly-His and His-Glu at their pl were capable of assuring pH control of a cathodic electrotransport formulation for at least 5 hours. This contrasts with the lack of stability observed with buffers such as phosphate or MOPS at the same or higher ionic strength.

Table 5 shows that Gly-His and His-Glu at their pl were capable of assuring pH control of anodic and cathodic electrotransport formulations for up to 24 hours.

**Table 3**

| **Buffer** | **Ionic strength (approx.)** | **Initial pH** | **Anodic pH after 5 h electrotransport** |
|---|---|---|---|
| Phosphate 10 mM | 0.015 | 6.9 | 3.7 to 5.5 |
| Phosphate 33 mM | 0.05 | 6.9 | 3.3 |
| Phosphate 100 mM | 0.15 | 6.8 | 6.5 |
| MOPS 20 mM | 0.01 | 7.0 | 4.1 |
| Gly-His 30 mM | 0.007 | 7.1 | 7.0 |
| Gly-His 100 mM | 0.02 | 7.4 | 7.3 |
| Gly-His 250 mM | 0.06 | 7.4 | 7.3 |
| His-Glu 100 mM | 0.015 | 5.1 | 5.2 |
| His-Glu 250 mM | 0.037 | 5.2 | 5.2 |

**Table 4**

| **Buffer** | **Ionic strength (approx.)** | **Initial pH** | **Cathodic pH after 5 h electrotransport** |
|---|---|---|---|
| Gly-His 100 mM | 0.02 | 7.6 | 7.3 |
| Gly-His 250 mM | 0.06 | 7.5 | 7.4 |
| His-Glu 100 mM | 0.015 | 5.3 | 5.0 |
| His-Glu 250 mM | 0.037 | 5.3 | 5.0 |
| Phosphate 10 mM | 0.015 | 6.9 | 4.2 |
| MOPS 20 mM | 0.01 | 7.0 | 5.4 |

**Table 5**

| **Buffer** | **Electrode** | **Initial pH** | **pH after 24 h electrotransport** |
|---|---|---|---|
| Gly-His 100 mM | Anode | 7.3 | 7.2 |
| Gly-His 250 mM | Anode | 7.2 | 7.2 |
| Gly-His 100 mM | Cathode | 7.6 | 7.3 |
| Gly-His 250 mM | Cathode | 6.9 | 7.0 |
| His-Glu 100 mM | Anode | 5.1 | 5.5 |
| His-Glu 250 mM | Anode | 5.2 | 5.4 |
| His-Glu 100 mM | Cathode | 5.3 | 5.4 |
| His-Glu 250 mM | Cathode | 5.3 | 5.2 |

### Example 3

The effect of the zwitterionic buffer Gly-His at pH 7.5 (pl) on the transdermal delivery of a synthetic radiolabeled decapeptide (DECAD) in the hairless guinea pig was evaluated. This model polypeptide drug is composed of D-amino acids and is excreted unchanged in urine. At pH 7.5 the net charge of DECAD is about +1.6.

The electrotransport systems used in this study had a silver foil anode and a silver chloride cathode. The anodic and cathodic reservoir gels each had a volume of approximately 350 mL and a skin contacting surface area of about 2 cm². The electrodes were connected to a DC power source which supplied a constant level of electric current of 0.100 mA/cm². The anodic reservoir comprised a skin-contacting gel containing the aqueous solution of the buffering agent and DECAD at the indicated concentrations and 3% hydroxyethyl cellulose (HEC) as well as 10% cholestyramine, a high molecular weight resin in chloride salt form which contributes chloride ions into the donor solution without introducing mobile cations which compete with the DECAD for delivery into the animal. The chloride ions from the cholestyramine resin are provided to react with any silver ions which are generated by electrochemical oxidation of the silver foil anode, thereby removing silver cations (ie, as potentially competing with the DECAD cations) from the donor solution. The cathodic reservoir contained a 0.15 M aqueous solution of sodium chloride in an HEC gel.

Experiments were performed in vivo in hairless guinea pigs. Three animals were used for each condition studied. The electrotransport systems were applied to and removed from the flank of the animals. The two reservoirs were generally spaced about 5 cm apart. The application site was wiped with water prior to system application. Flux was estimated over a 5 hour delivery period by analyzing the radioactive content of urine excreted for 48 hours. Donor reservoir gel pH was measured prior to application and after removal of the systems using a HORIBA compact pH meter (Cardy).

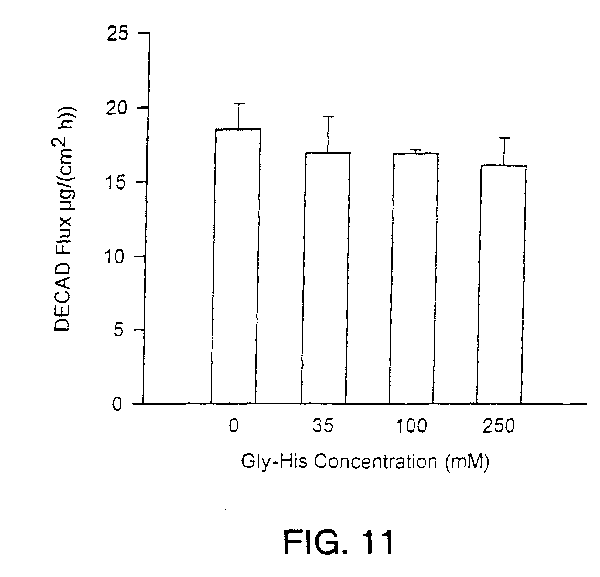

At concentrations up to 250 mM, Gly-His did not lower significantly the flux of the DECAD polypeptide as shown in Figure 11. Table 6 shows that in all experimental conditions tested, the dipeptide Gly-His was capable of assuring pH control.

**Table 6**

| **Gly-His conc. (mM)** | **DECAD conc. (mM)** | **Wearing time (h)** | **Initial pH** | **Final pH** |
|---|---|---|---|---|
| 100 | 0.5 | 5 | 7.3 | 7.2 |
| 10 | 5 | 5 | 6.9 | 7.0 |
| 30 | 5 | 5 | 7.1 | 7.0 |
| 100 | 5 | 5 | 7.4 | 7.3 |
| 250 | 5 | 5 | 7.4 | 7.3 |
| 100 | 5 | 24 | 7.4 | 7.3 |

### Example 4

The effect of the zwitterionic buffer Gly-His and His-Glu on pH stability of formulations containing small molecular weight drug-like compounds during electrotransport to hairless guinea pigs was studied. Trimethylammonium bromide (TMAB) was used as the cationic model drug and sodium methanesulfate (SMS) was used as the model anionic drug.

The electrotransport systems used in the study had a silver foil anode and a silver chloride cathode. The anodic and cathodic reservoir gels each had a volume of approximately 350 µL and a skin contacting surface area of about 2 cm². The electrodes were connected to a DC power source which supplied a constant level of electric current of 0.100 mA/cm². The anodic electrode assembly comprised a skin-contacting gel containing the aqueous solution of His-Glu 66 mM or Gly-His 45 mM and TMAB at 50 mM and 3% HEC. This formulation was separated from the silver anode by a Sybron ion exchange membrane. A gel containing 0.15 M sodium chloride (which acted as a chloride source) was placed between the silver anode and the ion exchange membrane. The cathodic electrode assembly comprised a skin-contacting gel containing the aqueous solution of His-Glu 66 mM or Gly-His 45 mM and SMS 50 mM and 3% HEC. The cathodic gel reservoir was separated from the silver chloride cathode by a Nafion ion exchange membrane. A gel containing 0.15M sodium chloride was placed between the cathode and the ion exchange membrane. The ionic strength of the skin-contacting gels was 60 mM.

Experiments were performed in vivo in hairless guinea pigs. Two animals were used for each condition studied. The electrotransport systems were applied to and removed from the backs of the animals. The two reservoirs were generally spaced about 5 cm apart. The application site was wiped with 70% isopropyl alcohol wipes prior to system application. Donor reservoir gel pH was measured prior to application and after removal of the systems using a HORIBA compact pH meter (Cardy).

Table 7 shows that in all experimental conditions tested, the dipeptides Gly-His and His-Glu provided good pH control.

**Table 7**

| **Drug** | **Buffer** | **Electrode** | **Initial pH** | **Final pH** |
|---|---|---|---|---|
| TMAB 50 mM | His-Glu 66 mM | Anode | 5.1 | 5.3 |
| SMS 50 mM | His-Glu 66 mM | Cathode | 5.2 | 6.0 |
| TMAB 50 mM | Gly-His 45 mM | Anode | 7.4 | 6.9 |
| SMS 50 mM | Gly-His 45 mM | Cathode | 7.5 | 7.9 |

## Claims

1. A transdermal electrotransport drug delivery device (10) having a reservoir (26,28) containing a reservoir formulation comprising an aqueous solution of a drug or an electrolyte and a dipeptide buffer, the dipeptide buffer comprising a polypeptide chain of 2 to 5 amino acids and having an isoelectric pH at which the dipeptide carries no net charge, the dipeptide having at least 2 pKa's which are separated by no more than about 3.5 pH units, the solution having a pH which is within 1.0 pH units of the isoelectric pH.

2. A transdermal electrotransport drug delivery device (10) as set forth in claim 1, wherein said reservoir is a drug-containing donor reservoir (26,28).

3. A transdermal electrotransport drug delivery device (10) as set forth in claim 1, wherein said reservoir is an electrolyte-containing counter reservoir (26,28).

4. The drug delivery device as set forth in any one of claims 1 to 3, wherein the isoelectric pH of the dipeptide is between about 3 and 10.

5. The drug delivery device as set forth in any one of claims 1 to 4, wherein the dipeptide is present in the reservoir formulation at a concentration of at least about 10 mM.

6. The drug delivery device as set forth in any one of claims 1 to 5, wherein the dipeptide includes at least one amino acid selected from the group consisting of his, tyr, arg, cys, lys, asp and glu.

7. The drug delivery device as set forth in claim 6, wherein the dipeptide includes histidine.

8. The drug delivery device as set forth in claim 7, wherein the dipeptide is gly-his.

9. The drug delivery device as set forth in any one of claims 1 to 4, wherein the dipeptide is selected from the group consisting of asp-asp, gly-asp, asp-his, glu-his, his-glu, his-asp, glu-arg, glu-lys, arg-glu, lys-glu, arg-asp, lys-asp, his-gly, his-ala, his-asn, his-citruline, his-gln, his-hydroxyproline, his-isoleucine, his-leu, his-met, his-phe, his-pro, his-ser, his-thr, his-trp, his-tyr, his-val, asn-his, thr-his, tyr-his, gln-his, phe-his, ser-his, citruline-his, trp-his, met-his, val-his, his-his, ile-his, hydroxyproline-his, leu-his, ala-his, gly-his, beta-alanylhistidine, pro-his, carnosine, anserine, tyr-arg, hydroxylysine-his, his-hydroxylysine, ornithine-his, his-lys, his-ornithine and lys-his.

10. The drug delivery device as set forth in any one of claims 1 to 9, wherein the drug comprises a polypeptide or a protein.

11. A method of buffering an aqueous solution of a drug or an electrolyte used for transdermal electrotransport delivery, comprising buffering the solution with a dipeptide buffer as defined in any one of claims 1, 4 or 6 to 9.

12. The method as set forth in claim 11, wherein the dipeptide is present in the solution at a concentration of at least about 10 mM.

13. The method as set forth in claim 11 or 12, wherein the drug comprises a polypeptide or a protein.

14. The method as set forth in any one of claims 11 to 13, wherein the solution is contained in a reservoir of a transdermal electrotransport drug delivery service.

## Patentansprüche

1. Elektrotransportvorrichtung (10) zur Arzneimittelabgabe über die Haut mit einem eine Vorratslösung enthaltenden Reservoir (26, 28), welche eine wässrige Lösung eines Wirkstoffs oder eines Elektrolyten und einen Dipeptidpuffer umfasst, wobei der Dipeptidpuffer eine Polypeptidkette mit 2 bis 5 Aminosäuren umfasst und einen isoelektrischen pH aufweist, bei dem das Dipeptid keine Nettoladung trägt, und das Dipeptid wenigstens 2 pKa-Werte aufweist, die sich um höchstens etwa 3,5 pH-Einheiten unterscheiden, und die Lösung einen pH aufweist, der sich maximal um 1,0 pH-Einheiten vom isoelektrischen pH unterscheidet.

2. Elektrotransportvorrichtung (10) zur Arzneimittelabgabe über die Haut nach Anspruch 1, wobei das Reservoir ein wirkstoffhaltiges Donorreservoir (26, 28) ist.

3. Elektrotransportvorrichtung (10) zur Arzneimittelabgabe über die Haut nach Anspruch 1, wobei das Reservoir ein Elektrolyt enthaltendes Gegenreservoir (26, 28) ist.

4. Arzneimittelabgabevorrichtung nach einem der Ansprüche 1 bis 3, wobei der isoelektrische pH des Dipeptids zwischen etwa 3 und 10 liegt.

5. Arzneimittelabgabevorrichtung nach einem der Ansprüche 1 bis 4, wobei das Dipeptid in der Vorratslösung in einer Konzentration von wenigstens etwa 10 mM vorliegt.

6. Arzneimittelabgabevorrichtung nach einem der Ansprüche 1 bis 5, wobei das Dipeptid wenigstens eine unter His, Tyr, Arg, Cys, Lys, Asp und Glu ausgewählte Aminosäure enthält.

7. Arzneimittelabgabevorrichtung nach Anspruch 6, wobei das Dipeptid Histidin enthält.

8. Arzneimittelabgabevorrichtung nach Anspruch 7, wobei das Dipeptid Gly-His ist.

9. Arzneimittelabgabevorrichtung nach einem der Ansprüche 1 bis 4, wobei das Dipeptid ausgewählt ist unter Asp-Asp, Gly-Asp, Asp-His, Glu-His, His-Glu, His-Asp, Glu-Arg, Glu-Lys, Arg-Glu, Lys-Glu, Arg-Asp, Lys-Asp, His-Gly, His-Ala, His-Asn, His-Citrulin, His-Gln, His-Hydroxypyrolin, His-Isoleucin, His-Leu, His-Met, His-Phe, His-Pro, His-Ser, His-Thr, His-Trp, His-Tyr, His-Val, Asn-His, Thr-His, Tyr-his, Gln-His, Phe-His, Ser-His, Citrulin-His, Trp-His, Met-His, Val-His, His-His, Ile-His, Hydroxyprolin-His, Leu-His, Ala-His, Gly-His, beta-Alanylhistidin, Pro-His, Carnosin, Anserin, Tyr-Arg, Hydroxylysin-His, His-Hydroxylysin, Ornithin-His, His-Lys, His-Ornithin und Lys-His.

10. Arzneimittelabgabevorrichtung nach einem der Ansprüche 1 bis 9, wobei der Wirkstoff ein Polypeptid oder ein Protein umfasst.

11. Verfahren zur Pufferung einer zur Abgabe durch Elektrotransport über die Haut verwendeten wässrigen Lösung eines Wirkstoffs oder eines Elektrolyten, bei dem man die Lösung mit einem Dipeptidpuffer gemäß Definition in einem der Ansprüche 1, 4 oder 6 bis 9 puffert.

12. Verfahren nach Anspruch 11, wobei das Dipeptid in der Lösung in einer Konzentration von wenigstens etwa 10 mM vorliegt.

13. Verfahren nach Anspruch 11 oder 12, wobei der Wirkstoff ein Polypeptid oder ein Protein umfasst.

14. Verfahren nach einem der Ansprüche 11 bis 13, wobei sich die Lösung in einem Reservoir einer Elektrotransportvorrichtung zur Arzneimittelabgabe über die Haut befindet.

## Revendications

1. Dispositif (10) d'administration de médicament par électrotransport transdermique comportant un réservoir (26, 28) contenant une formulation réservoir ayant une solution aqueuse d'un médicament ou un électrolyte et un tampon dipeptide, le tampon dipeptide comportant une chaîne polypeptidique de 2 à 5 acides aminés et ayant un pH isoélectrique auquel les dipeptides ne portent pas de charge nette, le dipeptide ayant au moins 2 pKa qui sont séparés par pas plus que 3,5 unités de pH environ, la solution ayant un pH qui se trouve, à 1,0 unité de pH près, au pH isoélectrique.

2. Dispositif (10) d'administration de médicament par électrotransport transdermique suivant la revendication 1, dans lequel le réservoir est un réservoir (26, 28) de donneur contenant un médicament.

3. Dispositif (10) d'administration de médicament par électrotransport transdermique suivant la revendication 1, dans lequel le réservoir est un contre-réservoir (26, 28) contenant un électrolyte.

4. Dispositif d'administration de médicament suivant l'une quelconque des revendications 1 à 3, dans lequel le pH isoélectrique du dipeptide est compris entre environ 3 et 10.

5. Dispositif d'administration de médicament suivant l'une quelconque des revendications 1 à 4, dans lequel le dipeptide est présent dans la formule du réservoir à une concentration d'au moins environ 10 mM.

6. Dispositif d'administration de médicament suivant l'une quelconque des revendications 1 à 5, dans lequel le dipeptide comporte au moins un acide aminé sélectionné dans le groupe constitué de his, tyr, arg, cys, lys, asp et glu.

7. Dispositif d'administration de médicament suivant la revendication 6, dans lequel le dipeptide comprend la histidine.

8. Dispositif d'administration de médicament suivant la revendication 7, dans lequel le dipeptide est le gly-his.

9. Dispositif d'administration de médicament suivant l'une quelconque des revendications 1 à 4, dans lequel le dipeptide est sélectionné dans le groupe constitué de asp-asp, gly-asp, asp-his, glu-his, his-glu, his-asp, glu-arg, glu-lys, arg-glu, lys-glu, arg-asp, lys-asp, his-gly, his-ala, his-asn, his-citruline, his-gln, his-hydroxyproline, his-isoleucine, his-leu, his-met, his-phe, his-pro, his-ser, his-thr, his-trp, his-tyr, his-val, asn-his, thr-his, tyr-his, gln-his, phe-his, ser-his, citruline-his, trp-his, met-his, val-his, his-his, ile-his, hydroxyproline-his, leu-his, ala-his, gly-his, beta-alanylhistidine, pro-his, camosine, anserine, tyr-arg, hydroxylysine-his, his-hydroxylysine, omithine-his, his-lys, his-ornithine et lys-his.

10. Dispositif d'administration de médicament suivant l'une quelconque des revendications 1 à 9, dans lequel le médicament comporte un polypeptide ou une protéine.

11. Procédé pour tamponner une solution aqueuse d'un médicament ou d'un électrolyte utilisé pour l'administration transdermique par électrotransport, consistant à tamponner la solution avec un tampon dipeptide tel que défini dans l'une quelconque des revendications 1, 4 ou 6 à 9.

12. Procédé suivant la revendication 11, dans lequel le dipeptide est présent dans la solution à une concentration d'au moins environ 10 mM.

13. Procédé suivant la revendication 11 ou 12, dans lequel le médicament comporte un polypeptide ou une protéine.

14. Procédé suivant l'une quelconque des revendications 11 à 13, dans lequel la solution est contenue dans un réservoir d'un dispositif d'administration transdermique de médicament par électrotransport.
